(19) Europäisches Patentamt
European Patent Office
Office européen des brevets

(11) Veröffentlichungsnummer : **0 053 306**
**B1**

(12) # EUROPÄISCHE PATENTSCHRIFT

(45) Veröffentlichungstag der Patentschrift : **17.10.84**

(51) Int. Cl.³ : **C 07 D213/68**, A 01 N 43/40// C07D213/61

(21) Anmeldenummer : **81109617.1**

(22) Anmeldetag : **11.11.81**

(54) **Pyridinderivate, ihre Herstellung und Verwendung.**

(30) Priorität : **28.11.80 DE 3044856**

(43) Veröffentlichungstag der Anmeldung : **09.06.82 Patentblatt 82/23**

(45) Bekanntmachung des Hinweises auf die Patenterteilung : **17.10.84 Patentblatt 84/42**

(84) Benannte Vertragsstaaten : **AT BE CH DE FR GB IT LI LU NL**

(56) Entgegenhaltungen :
**EP-A- 0 003 877**
**EP-A- 0 021 613**
**DE-A- 1 542 736**
**DE-A- 2 223 894**
**US-A- 4 218 456**
**US-A- 4 223 033**
**US-A- 4 228 172**

(73) Patentinhaber : **CELAMERCK GmbH & Co. KG**
**Binger Strasse 173**
**D-6507 Ingelheim am Rhein (DE)**

(72) Erfinder : **Schröder, Ludwig, Dr.**
**Frankenstrasse 6**
**D-6507 Ingelheim am Rhein (DE)**
Erfinder : **Stransky, Werner, Dr. Dipl.-Chem.**
**Im Hippel 24**
**D-6535 Gau-Algesheim (DE)**
Erfinder : **Mengel, Rudolf, Dr. Dipl.-Chem.**
**Schützenpfad 22**
**D-6507 Ingelheim am Rhein (DE)**
Erfinder : **Linden, Gerbert, Dr. Dipl.-Landw.**
**Turnierstrasse 44**
**D-6507 Ingelheim am Rhein (DE)**
Erfinder : **Lust, Sigmund, Dr.**
**Klappacher Strasse 2f**
**D-6100 Darmstadt (DE)**

**EP 0 053 306 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft neue Pyridinderivate der Formel

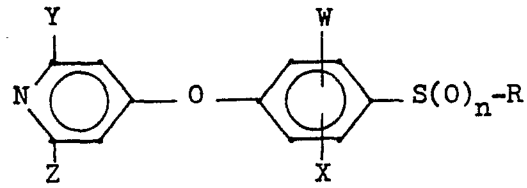

(I)

in der

n für 0, 1 oder 2,

R für einen gesättigten, gegebenenfalls durch ein oder mehrere Fluor- oder Chloratome substituierten, oder für einen ungesättigten aliphatischen Rest mit bis zu 4 C-Atomen, einen Benzylrest oder einen chlor- oder methyl-substituierten Benzylrest,

W und X, die gleich oder verschieden sein können, für Wasserstoff, Cl, F, Br, J oder $C_1$-$C_4$-Alkyl,

Y für Wasserstoff, Cl, F, Br oder $C_1$-$C_4$-Alkyl und

Z für Cl oder F stehen,

die Herstellung der neuen Verbindungen und ihre Verwendung als biozide, insbesondere herbizide Wirkstoffe. Als bevorzugte Substituentenbedeutungen sind zu nennen R gleich Methyl oder halogeniertes Methyl, z. B. $CH_2Cl$, $CF_3$ ; W und X Wasserstoff, Methyl oder Chlor ; Y Wasserstoff, Chlor oder Methyl ; Z Chlor. Soweit die aliphatischen Reste 3 oder 4 C-Atome enthalten, können sie verzweigt oder unverzweigt sein.

Aus der DE-OS 15 42 736 sind aryloxysubstituierte Pyridine bekannt, die der allgemeinen Formel

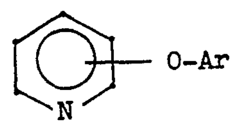

entsprechen. Diese Verbindungen, die im Pyridinring keine zusätzlichen Substituenten aufweisen, beispielsweise die besonders herausgestellte Verbindung

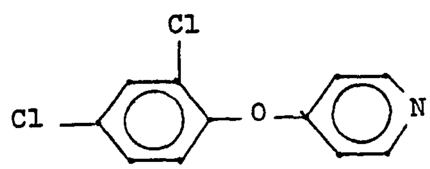

befriedigen in ihrer Wirkung nicht.

Die erfindungsgemäßen Verbindungen zeichnen sich demgegenüber durch eine starke Wirkung gegen zahlreiche, insbesondere dikotyle Unkräuter, z. B. auch schwer bekämpfbare wie Galium aparine, Veronica hederifolia aus. Die Anwendung erfolgt bevorzugt vor dem Auflaufen. Von erheblichem Nutzen ist auch die Möglichkeit, für die Praxis bedeutsame Wirkungslücken vieler sonst sehr bewährter Handelsprodukte zu schließen, indem man die neuen Wirkstoffe gemeinsam mit ihnen anwendet.

Kombinationspartner dieser Art sind beispielsweise : Chlortoluron und verwandte Harnstoffderivate, Terbutryn und verwandte Triazinderivate, Trifluralin und verwandte Derivate, Alachlor, Dimethachlor.

Die neuen Verbindungen werden in an sich bekannter Weise hergestellt.

Man setzt dazu ein Pyridinderivat der Formel

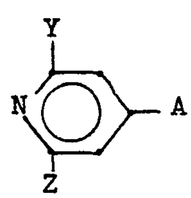

(II)

(A gleich Halogen, insbesondere Chlor, Fluor oder $NO_2$)
mit einem Phenol oder Phenolat der Formel

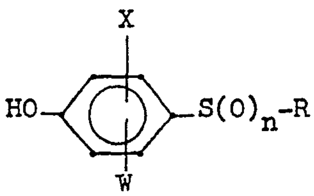 bzw. 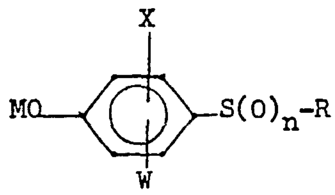

(IIIa) (IIIb)

(M gleich 1 Äquivalent eines Kations, vorzugsweise $Na^{\oplus}$, $K^{\oplus}$, 1/2 $Ca^{2+}$) um und oxidiert gewünschtenfalls solche Reaktionsprodukte, in denen n 0 oder 1 bedeutet, zu den höheren Oxidationsstufen (mit n gleich 1 bzw. 2).

Die Umsetzung der Verbindungen II und IIIa bzw. IIIb erfolgt bei Temperaturen zwischen der Umgebungstemperatur und etwa 160 °C. Man arbeitet vorzugsweise in polaren Lösungsmitteln wie Aceton, Butan-2-on, Acetonitril, Dimethylformamid, Dimethylsulfoxid bei der Siedetemperatur des Reaktionsgemischs. Bei der Umsetzung der Phenole gibt man säurebindende Mittel, z. B. Alkalicarbonate, etwa $Na_2CO_3$, $K_2CO_3$, oder Alkali- oder Erdalkalihydroxide (NaOH, KOH, $Ca(OH)_2$) zu. Um eine günstige Ausbeute zu erzielen, kann es vorteilhaft sein, das Phenol bzw. dessen Salze und/oder das säurebindende Mittel im Überschuß einzusetzen.

Für die Oxidation der Mercaptoverbindungen zu den entsprechenden Sulfinyl- und Sulfonylverbindungen bzw. der Sulfinylverbindungen zu den Sulfonylverbindungen bedient man sich der für diese Reaktion üblichen Oxidationsmittel. Für die Oxidation der Thioäther zu den Sulfinylverbindungen seien genannt Salpetersäure, 1 Äquivalent Wasserstoffperoxid, Distickstofftetroxid oder Brom, Chlor, Jod.

Für die Oxidation der Thioäther bzw. der Sulfinylverbindungen zu den Sulfonylverbindungen werden entsprechend erhöhte Mengen Wasserstoffperoxid, Kaliumhydrogenpersulfat oder Kaliumpermanganat verwendet.

Die Ausgangsstoffe der Formeln II, IIIa und IIIb sind bekannt oder können analog den bekannten Verbindungen erhalten werden.

Die Verbindungen der Formel I werden für die Anwendung im Pflanzenschutz mit gebräuchlichen Hilfs- und/oder Trägerstoffen zu üblichen Formulierungen verarbeitet.

Die Aufwandmengen bei der Anwendung als Herbizide liegen für die neuen Verbindungen im allgemeinen zwischen etwa 0,05 und 2 kg, vorzugsweise 0,1 und 1 kg, pro Hektar, je nach der verwendeten Substanz und dem zu bekämpfenden Unkraut. In Kombination mit anderen Herbiziden genügen im allgemeinen noch geringere Mengen (bis hinunter zu etwa 0,05 kg/ha) der erfindungsgemäßen Verbindungen. Die nachstehenden Zubereitungen stellen Beispiele für die Formulierung der neuen herbiziden Mittel dar (Angaben in Gewichtsprozent) :

a) Suspensionspulver 1

25 % Wirkstoff gemäß Formel I
55 % Kaolin
9 % Ligninsulfonat als Dispergiermittel
1 % Natriumtetrapropylenbenzolsulfonat als Netzmittel

b) Suspensionspulver 2

80 % Wirkstoff gemäß Formel I
8 % Calciumligninsulfonat
5 % kolloidale Kieselsäure
5 % Natriumsulfat
2 % Natriumdiisobutylnaphthalinsulfonat

c) Emulsionskonzentrat

40 % Wirkstoff gemäß Formel I
25 % Shellsol A (flüssiges Gemisch aromatischer Kohlenwasserstoffe)
25 % N-Methylpyrrolidon
10 % Emulsogen I 40 (anionenaktiver Emulgator)

Die unter a) bis c) angegebenen Konzentrate werden für die Anwendung mit Wasser auf etwa 0,05 bis 5 Gewichtsprozent verdünnt.

Die Wirkung der neuen Verbindungen wurde im Gewächshausversuch mit der Aufwandmenge 4 kg/ha geprüft (Vorauflaufwerte).

Als Vergleich diente A (= 4-(2,4-Dichlorphenoxy)pyridin nach der DE-OS 15 43 736).

Die Bewertung erfolgte nach dem neunstufigen Bonitierungsschlüssel, wobei 1 = 100 % Wirkung, 9 = keine Wirkung bedeutet.

| Verbindung | SIN * | LYC * |
|---|---|---|
| A (Stand der Technik) | 9 | 9 |
| erfindungsgemäß | | |
| Beispiel 1 | 1 | 1 |
| Beispiel 3 | 1 | 1 |
| Tab. Nr. 13 | 2 | 1 |
| Tab. Nr. 21 | 1 | 1 |
| Tab. Nr. 30 | 1 | 1 |

*SIN = Sinapis alba
LYC = Lycopersicum esculentum

Mit der guten Wirksamkeit der neuen Verbindungen verbindet sich eine gute Verträglichkeit in zahlreichen Kulturen (z. B. Hafer, Weizen, Gerste, Kartoffeln, Soja, Baumwolle, Erbse), wodurch ein selektiver Einsatz ermöglicht wird.

Die Anwendung kann sowohl vor wie auch nach dem Auflaufen erfolgen.

Für Kombinationen, vor allem für die Nachauflaufbehandlung, eignen sich besonders

1. Wuchsstoffherbizide, etwa 2,4-DP, MCPA, CMPP ;
2. Kontaktherbizide, etwa Ioxynil, Bromoxynil, Bentazon, Bromphenoxin, Dinoterb ;
3. Grasherbizide, etwa Isoproturon, Diclofopmethyl.

Die folgenden Beispiele sollen die erfindungsgemäßen Herstellverfahren näher erläutern, ohne sie zu beschränken :

## Herstellungsbeispiele

### Beispiel 1

2-Chlor-4-(3-methyl-4-methylmercapto-phenoxy)-pyridin

Eine Lösung von 17 g (0,1 Mol 3-Methyl-4-methylmercaptophenol sowie 15,9 g (0,1 Mol) 2-Chlor-4-nitropyridin in 150 ml Acetonitril wird mit 13,8 g (0,1 Mol) $K_2CO_3$ versetzt und 3 Stunden unter Rückfluß gerührt. Danach wird abgekühlt und auf 300 ml Wasser gegossen. Das abgeschiedene Öl wird abgetrennt, in Methylenchlorid aufgenommen und mit verdünnter Natronlauge und Wasser gewaschen. Danach wird die organische Lösung über $Na_2SO_4$ getrocknet und eingeengt. Das verbleibende Öl wird aus Hexan kristallisiert.

Ausbeute : 23 g (86 % der Theorie) Fp. : 45-46 °C.
Analyse : $C_{13}H_{12}ClNOS$ (265,76).
berechnet : C 58,75 %, H 4,55 %, N 5,27 %.
gefunden : C 59,02 %, H 4,60 %, N 4,98 %.

### Beispiel 2

2-Chlor-4-(2,5-dichlor-4-methylsulfinyl-phenoxy)-pyridin

32,6 g (0,1 Mol) 2-Chlor-4-(2,5-dichlor-4-methyl-mercaptophenoxy)-pyridin (hergestellt analog Beispiel 1) werden in 100 ml Eisessig suspendiert. Man erwärmt auf 45°-50 °C und tropft 11 ml (0,11 Mol) 30 %iges $H_2O_2$ so ein, daß die Innentemperatur 50 °C nicht überschreitet. Anschließend wird noch 3 Stunden bei 50 °C gerührt, dann abgekühlt und auf 300 ml Wasser gegossen. Man rührt bis zur vollständigen Kristallisation, saugt scharf ab, wäscht mit Wasser, trocknet und kristallisiert aus Essigester um.

Ausbeute : 20,2 g (60 % der Theorie), Fp. : 117-120 °C.
Analyse : $C_{12}H_8Cl_3NO_2S$ (336,63).
berechnet : C 42,8 %, H 2,39 %, N 4,16 %.
gefunden : C 42,84 %, H 2,54 %, N 4,08 %.

Beispiel 3

2-Chlor-4-(2,5-dichlor-4-methylsulfonyl-phenoxy)-pyridin

32,06 g (0,1 Mol) 2-Chlor-4-(2,5-dichlor-4-methyl-mercaptophenoxy)-pyridin (hergestellt analog Beispiel 1) werden in 100 ml Eisessig suspendiert. Man erwärmt unter Rühren auf 80 °C und tropft 22 ml (0,22 Mol) 30 %iges $H_2O_2$ so ein, daß die Innentemperatur 90 °C nicht überschreitet. Danach wird noch weitere 4 Stunden bei dieser Temperatur gerührt. Anschließend kühlt man ab und gießt auf 300 ml Wasser. Der gebildete Niederschlag wird abgesaugt, mit Wasser gewaschen, getrocknet und aus Essigester umkristallisiert.

Ausbeute : 28,6 g (81 % der Theorie), Fp. : 155-157 °C.
Analyse : $C_{12}H_8Cl_3NO_3S$ (352,63).
berechnet : C 40,85 %, H 2,29 %, N 3,96 %.
gefunden : C 40,97 %, H 2,48 %, N 4,08 %.

Beispiel 4

2-Chlor-6-methyl-(2,5-dichlor-4-methylmercaptophenoxy)-pyridin

Man erhitzt eine Lösung von 25,5 g (0,1 Mol) Kalium-2,5-dichlor-4-methylmercapto-phenolat und 16,2 g (0,1 Mol) 2,4-Dichlor-6-methyl-pyridin in 100 ml Dimethylformamid 5 Stunden unter Rückfluß. Danach wird abgekühlt und auf 300 ml Wasser gegossen.

Der ausgefallene Niederschlag wird abgesaugt, mit Wasser gewaschen und aus Isopropanol umkristallisiert.

Ausbeute : 13 g (39 % der Theorie) Fp. : 145-148 °C.
Analyse : $C_{13}H_{10}Cl_3NOS$ (334,65).
berechnet : C 46,65 %, H 3,01 %, N 4,19 %.
gefunden : C 46,98 %, H 3,17 %, N 4,02 %.

Beispiel 5

2,6-Dichlor-4-(2,5-dichlor-4-methylmercapto-phenoxy)-pyridin

Man löst 19,3 g (0,1 Mol) 2,6-Dichlor-4-nitro-pyridin und 23,1 g (0,11 Mol) 2,5-Dichlor-4-methylmercaptophenol in 100 ml Aceton, fügt 13,8 g (0,1 Mol) $K_2CO_3$ hinzu und rührt 3 Stunden unter Rückfluß. Danach wird in 500 ml Wasser eingerührt und der entstandene Niederschlag abgesaugt.

Ausbeute : 32,5 g (91,5 % der Theorie) Fp. : 144-146 °C.
Analyse : $C_{12}H_7Cl_4NOS$ (355,08).
berechnet : C 40,59 %, H 1,99 %, N 3,95 %
gefunden : C 40,78 %, H 2,30 %, N 3,85 %.

Herstellung des Ausgangsmaterials für Beispiel 5 : 2,6-Dichlor-4-nitropyridin

Man erhitzt eine Lösung von 20,9 g (0,1 Mol) 2,6-Dichlor-4-nitro-pyridin-1-oxyd (J. Chem. Soc. B *1967*, 1235) in 100 ml Äthylenchlorid zum Sieden und läßt dazu 27,5 g (0,2 Mol) Phosphortrichlorid einfließen. Die erhaltene Lösung wird weitere 8 Stunden unter Rückfluß gerührt und danach im Vakuum eingeengt. Der verbleibende ölige Rückstand wird auf Eiswasser gegeben. Der entstandene kristalline Rückstand wird abgesaugt und mit Wasser neutral gewaschen.

Ausbeute : 17,5 g (91 % der Theorie), Fp. : 98-99 °C.
Analyse : $C_5H_2Cl_2N_2O_2$ (193).
berechnet : C 31,11 %, H 1,04 %, N 14,52 %.
gefunden : C 31,31 %, H 1,30 %, N 14,38 %.

Beispiel 6

2-Fluor-6-methyl-4-(2,5-dichlor-4-methylmercaptophenoxy)pyridin

12,9 g (0,1 Mol) 2,4-Difluor-6-methyl-pyridin und 20,7 g (0,1 Mol) 2,5-Dichlor-4-methylmercaptophenol werden zusammen mit 13,8 g (0,1 Mol) Kaliumcarbonat 10 Stunden bei 100 °C in 100 ml Dimethylformamid gerührt. Die Lösung wird nun auf 500 ml gegossen, und der entstandene Niederschlag wird abgesaugt und mit Wasser gewaschen. Man nimmt in Methylenchlorid auf, trocknet über Natriumsulfat und engt ein. Das erhaltene Öl wird über eine Kieselgelsäule (Laufmittel Ethylenchlorid) gereinigt und man erhält 16,3 g (57 % der Theorie) des gewünschten Ethers ;

Fp. : 93-95 °C.

Entsprechend den vorstehenden Beispielen erhält man :

2,6-Difluor-4-(2,5-dichlor-4-methylmercaptophenoxy)-pyridin, Fp. 96-98 °C ;
2,6-Difluor-4-(2,5-dichlor-4-methylsulfinylphenoxy)-pyridin, Fp. 138-140 °C ;
2-Fluor-6-methyl-4-(2,5-dichlor-4-methylmercaptophenoxy)-pyridin, Fp. 93-95 °C.

Entsprechend den Beispielen 1 bis 6 können auch die Verbindungen der nachstehenden Tabelle erhalten werden :

Verbindungen der Formel

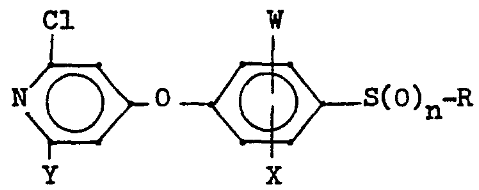

| Nr. | Y | W | X | n | R | Fp.(Kp./mbar) |
|---|---|---|---|---|---|---|
| 1 | H | H | H | 0 | $CH_3$ | 74-65°C |
| 2 | H | H | H | 1 | $CH_3$ | (199-201°C/0,02) |
| 3 | H | H | H | 2 | $CH_3$ | 120-122°C |
| 4 | H | $2\text{-}CH_3$ | H | 0 | $CH_3$ | (155-160°C/0,01) |
| 5 | H | $2\text{-}CH(CH_3)(C_2H_5)$ | H | 0 | $CH_3$ | (175-180°C/0,01) |
| 6 | H | 2-Cl | 5-Cl | 0 | $CH_3$ | 108-110°C |
| 7 | H | 3-Cl | 5-Cl | 1 | $CH_3$ | 83-86°C |
| 8 | H | 3-Cl | 5-Cl | 2 | $CH_3$ | 118-121°C |
| 9 | H | 3-Cl | 5-Cl | 0 | $CH_2\text{-}C_6H_5$ | 72-76°C |
| 10 | H | 3-Cl | 5-Cl | 1 | $CH_2\text{-}C_6H_5$ | 110-113°C |
| 11 | H | 3-Cl | 5-Cl | 2 | $CH_2C_6H_5$ | 166-169°C |
| 12 | H | 3-Cl | 5-Cl | 0 | $CH_2\text{-}CH{=}CH_2$ | (193-196°C/0,04) |
| 13 | H | 3-Cl | H | 0 | $CH_3$ | 82-84°C |
| 14 | H | 3-Cl | H | 1 | $CH_3$ | 85-88°C |
| 15 | H | 3-Cl | H | 2 | $CH_3$ | 125-129°C |
| 16 | H | $3\text{-}CH_3$ | $5\text{-}CH_3$ | 0 | $CH_3$ | (192-196°C/0,08) |
| 17 | $CH_3$ | H | H | 0 | $CH_3$ | 68-72°C |
| 18 | $CH_3$ | H | H | 0 | $CH_3$ | (180-185°C/0,002) |
| 19 | $CH_3$ | 3-Cl | H | 0 | $CH_3$ | 109-111°C |
| 20 | $CH_3$ | 3-Cl | H | 1 | $CH_3$ | 129-130°C |
| 21 | $CH_3$ | 3-Cl | H | 2 | $CH_3$ | 118-120°C |
| 22 | $CH_3$ | H | H | 2 | $CH_3$ | 119-121°C |
| 23 | $CH_3$ | 3-Cl | H | 0 | $C_4H_9$ | 81-84°C |
| 24 | $CH_3$ | 2-Cl | 5-Cl | 1 | $CH_3$ | 144-146°C |
| 25 | $CH_3$ | 2-Cl | 5-Cl | 2 | $CH_3$ | 164-167°C |
| 26 | $CH_3$ | 3-Cl | 5-Cl | 0 | $CH_3$ | 121-123°C |
| 27 | $CH_3$ | 3-Cl | 5-Cl | 0 | $CH_2\text{-}CH{=}CH_2$ | 103-105°C |
| 28 | Cl | H | H | 0 | $CH_3$ | 86-88°C |
| 29 | Cl | 2-Cl | 5-Cl | 1 | $CH_3$ | 175-178°C |
| 30 | Cl | 2-Cl | 5-Cl | 2 | $CH_3$ | 204-206°C |
| 31 | Cl | 2-Cl | 5-Cl | 0 | $CH_2Cl$ | Öl |
| 32 | Cl | 2-Cl | 5-Cl | 0 | $CF_3$ | Öl |

**Ansprüche**

1. Verbindungen der Formel

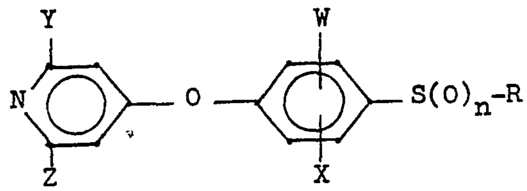

(I)

in der

n für 0, 1 oder 2,

R für einen gesättigten, gegebenenfalls durch ein oder mehrere Fluor- oder Chloratome substituierten, oder für einen ungesättigten aliphatischen Rest mit bis zu 4 C-Atomen, einen Benzylrest oder einen Chlor- oder methylsubstituierten Benzylrest,

W und X, die gleich oder verschieden sein können, für Wasserstoff, Cl, F, Br, J oder $C_1$-$C_4$-Alkyl,

Y für Wasserstoff, Cl, F, Br oder $C_1$-$C_4$-Alkyl und

Z für Cl oder F stehen.

2. Biozide Mittel, gekennzeichnet durch einen Gehalt an einer Verbindung nach Anspruch 1.

3. Verwendung von Verbindungen nach Anspruch 1 bei der Bekämpfung von Unkräutern.

4. Verfahren zur Herstellung von Verbindungen nach Anspruch 1, dadurch gekennzeichnet, daß man ein Pyridinderivat der Formel

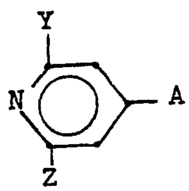

(II)

worin Y und Z die in Anspruch 1 angegebene Bedeutung haben und A Halogen, insbesondere Chlor oder Fluor, oder $NO_2$ ist, mit einem Phenol oder Phenolat der Formel

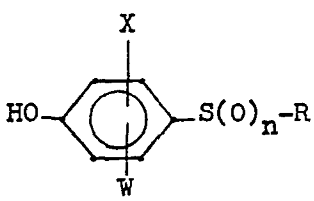

bzw.

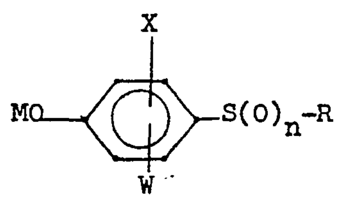

(IIIa) (IIIb)

worin n, R, W und X die in Anspruch 1 angegebene Bedeutung haben und M gleich 1 Äquivalent eines Kations ist, umsetzt, wobei im Fall des Phenols ein säurebindendes Mittel zugesetzt wird, und daß man gegebenenfalls solche Reaktionsprodukte, in denen n 0 oder 1 bedeutet, mit dafür gebräuchlichen Oxidationsmitteln zu den höheren Oxidationsstufen oxidiert.

5. Verbindungen nach Anspruch 1, dadurch gekennzeichnet, daß R Methyl oder halogeniertes Methyl, W, X und Y, die gleich oder verschieden sein können, Wasserstoff, Chlor oder Methyl bedeuten.

6. Herbizide Mittel, gekennzeichnet durch einen Gehalt an einer Verbindung nach Anspruch 5.

7. Verwendung einer Verbindung nach Anspruch 5 bei der Bekämpfung von Unkräutern.

8. Herbizide Mittel, dadurch gekennzeichnet, daß sie als aktive Bestandteile neben einer Verbindung nach Anspruch 1 zusätzlich Chlortoluron oder einen verwandten Harnstoff, Terbutryn oder ein verwandtes Triazinderivat, Trifluralin oder eine verwandte Verbindung, Alachlor oder Dimethachlor, 2,4-DP, MCPA, CMPP, Ioxynil, Bromoxynil, Bentazon, Bromphenoxim, Dinoterb, Isoproturon oder Dichlofopmethyl enthalten.

9. Verwendung einer Verbindung nach Anspruch 1 in Kombination mit Chlortoluron oder einem verwandten Harnstoff, Terbutryn oder einem verwandten Triazinderivat, Trifluralin oder einer verwandten Verbindung, Alachlor oder Dimethachlor, 2,4-DP, MCPA, CMPP, Ioxynil, Bromoxynil, Bentazon, Bromphenoxim, Dinoterb, Isoproturon oder Dichlofopmethyl bei der Bekämpfung von Unkräutern.

**Claims**

1. Compounds of formula

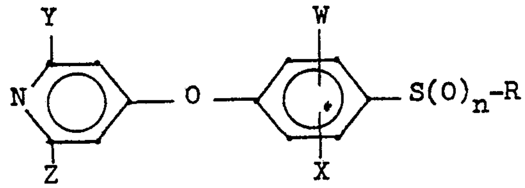

(I)

wherein

n represents 0, 1 or 2,

R represents a saturated aliphatic group with up to 4 carbon atoms optionally substituted by one or more fluorine or chlorine atoms or an unsaturated aliphatic group with up to 4 carbon atoms, a benzyl group or a chlorine- or methyl-substituted benzyl group,

W and X, which may be the same or different, represent hydrogen, Cl, F, Br, I or $C_{1-4}$ alkyl,

Y represents hydrogen, Cl, F, Br or $C_{1-4}$ alkyl and

Z represents Cl or F.

2. Biocidal compositions characterised in that they contain a compound as claimed in claim 1.

3. Use of compounds as claimed in claim 1 for combating weeds.

4. Process for the preparation of compounds as claimed in claim 1, characterised in that a pyridine derivative of formula

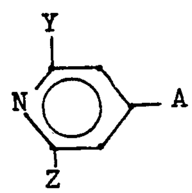

(II)

wherein Y and Z are defined as in claim 1 and A is halogen particularly chlorine or fluorine, or $NO_2$, is reacted with a phenol or phenoxide of formula

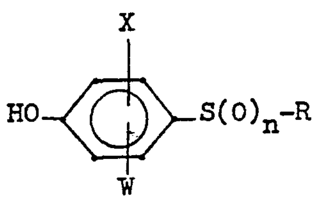

or

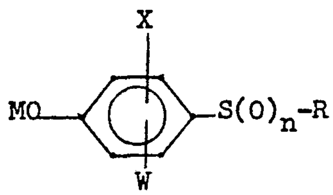

(IIIa) (IIIb)

wherein N, R, W and X are defined as in claim 1 and M is 1 equivalent of a cation, and in the case of the phenol, an acid-binding agent is added and, if desired, reaction products wherein n represents 0 or 1 are oxidised with the conventional oxidising agents to give the higher oxidation stages.

5. Compounds as claimed in claim 1, characterised in that R represents methyl or halogenated methyl, and W, X and Y, which may be the same or different, represent hydrogen, chlorine or methyl.

6. Herbicidal compositions, characterised in that they contain a compound as claimed in claim 5.

7. Use of a compound as claimed in claim 5 for combating weeds.

8. Herbicidal compositions, characterised in that they contain, as active ingredients, in addition to a compound as claimed in claim 1, chlortoluron or a related urea, terbutryn or a related triazine derivative, trifluralin or a related compound, alachlor or dimethachlor, 2,4-DP, MCPA, CMPP, ioxynil, bromoxynil, bentazon, bromphenoxim, dinoterb, isoproturon or dichlofopmethyl.

9. Use of a compound as claimed in claim 1 in conjunction with chlortoluron or a related urea, terbutryn or a related triazine derivative, trifluralin or a related compound, alachlor or dimethachlor, 2,4-DP, MCPA, CMPP, ioxynil, bromoxynil, bentazon, bromphenoxim, dinoterb, isoproturon or dichlofopmethyl for combating weeds.

**Revendications**

1. Composés de formule

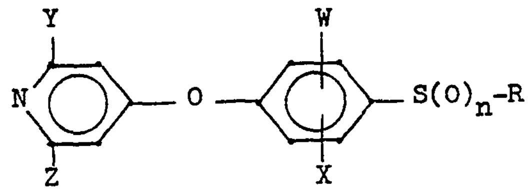

(I)

dans laquelle

n remplace 0, 1 ou 2,

R remplace un radical aliphatique avec jusqu'à 4 atomes de C, saturé, éventuellement substitué par un ou plusieurs atomes de fluor ou de chlore, ou insaturé, un radical benzyle ou un radical benzyle chloro- ou méthyl-substitué,

W et X, qui peuvent être identiques ou différents, remplacent l'hydrogène, Cl, F, Br, I ou alcoyle en $C_1$-$C_4$,

Y remplace l'hydrogène, Cl, F, Br ou alcoyle en $C_1$-$C_4$, et

Z remplace Cl ou F.

2. Agent biocide, caractérisé par une teneur en un composé selon la revendication 1.

3. Utilisation de composés selon la revendication 1 dans la lutte contre les mauvaises herbes.

4. Procédé pour la préparation de composés selon la revendication 1, caractérisé en ce qu'on fait réagir un dérivé de pyridine de formule

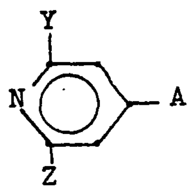

(II)

dans laquelle Y et Z ont la signification indiquée dans la revendication 1, et A est halogène, en particulier chlore ou fluor, ou $NO_2$, avec un phénol ou phénolate de formule

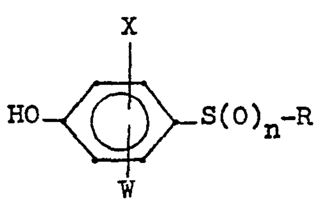

ou

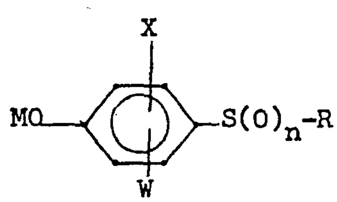

(IIIa) (IIIb)

où n, R, W et X ont la signification indiquée dans la revendication 1 et M est tel qu'un équivalent d'un cation, en ajoutant, dans le cas du phénol, un agent fixant les acides, et en ce qu'éventuellement, on oxyde de tels produits de réaction dans lesquels n signifie 0 ou 1, au moyen d'agents d'oxydation usuels pour ce faire, en les degrés d'oxydation supérieurs.

5. Composés selon la revendication 1, caractérisés en ce que R représente méthyle ou méthyle halogéné, W, X et Y qui peuvent être identiques ou différents représentent hydrogène, chlore ou méthyle.

6. Agent herbicide, caractérisé en une teneur en un composé selon la revendication 5.

7. Utilisation d'un composé selon la revendication 5 dans la lutte contre les mauvaises herbes.

8. Agents herbicides, caractérisés en ce qu'ils contiennent en tant que constituant actif, à côté d'un composé selon la revendication 1, en outre du chlortoluron ou une urée apparentée, de la terbutryne ou un dérivé de triazine apparenté, de la trifluraline ou un composé apparenté, de l'alachlor ou di diméthachlor, du 2,4-DP, du MCPA, du CMPP, de l'ioxynil, du bromoxynil, de la bentazone, de la bromophénoxime, du dinoterb, de l'isoproturon ou du dichlofopméthyle.

9. Utilisation d'un composé selon la revendication 1 en combinaison avec du chlortoluron ou avec une urée apparentée, de la terbutryne ou un dérivé de triazine apparenté, de la trifluraline ou un composé apparenté, de l'alachlor ou du diméthachlor, du 2,4-DP, du MCPA, du CMPP, de l'ioxynil, du bromoxynil, de la bentazone, de la bromophénoxime, du dinoterb, de l'isoproturon ou du dichlofopméthyl dans la lutte contre les mauvaises herbes.